(19) Europäisches Patentamt
European Patent Office
Office européen des brevets

(11) Veröffentlichungsnummer : **0 116 517**
**B1**

(12) # EUROPÄISCHE PATENTSCHRIFT

(45) Veröffentlichungstag der Patentschrift : **18.06.86**

(51) Int. Cl.[4] : **C 07 C149/36**, C 07 D307/64, C 09 K 15/14, C 08 K 5/37

(21) Anmeldenummer : **84810054.1**

(22) Anmeldetag : **30.01.84**

(54) **Ester oder Anhydride von substituierten 4-(Hydroxyphenylthio)bernsteinsäuren.**

(30) Priorität : **03.02.83 US 463176**

(43) Veröffentlichungstag der Anmeldung : **22.08.84 Patentblatt 84/34**

(45) Bekanntmachung des Hinweises auf die Patenterteilung : **18.06.86 Patentblatt 86/25**

(84) Benannte Vertragsstaaten : **BE DE FR GB IT NL**

(56) Entgegenhaltungen :
**EP-A- 0 079 855**
**NL-A- 7 207 808**
**US-A- 3 078 290**

(73) Patentinhaber : **CIBA-GEIGY AG**
**Klybeckstrasse 141**
**CH-4002 Basel (CH)**

(72) Erfinder : **Spivack, John D.**
**1 Blue Jay Street**
**Spring Valley New York 10977 (US)**
Erfinder : **Pastor, Stephen D.**
**112 Union Road**
**Spring Valley New York 10977 (US)**

**EP 0 116 517 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Mercaptophenolderivate, deren Verwendung als Stabilisatoren sowie das damit stabilisierte organische Material.

Organische polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Ueberraschenderweise ist jetzt gefunden worden, dass Ester bzw. Anhydride von Hydroxyphenylthiobernsteinsäure eine ungewöhnliche Kombination wünschenswerter Eigenschaften besitzen, wodurch diese Stoffe zu besonders effektiven und nützlichen Stabilisatoren werden. Die erfindungsgemässen Verbindungen erweisen sich als besonders nützliche Stabilisatoren für schlagfestes Polystyrol, Elastomere wie Polybutadien oder Styrol-Butadien-Elastomere, bei denen der Erhalt der Elastizität, die Verhinderung von Vernetzungsreaktionen von Verfärbung, von Geruchsbildung und von Ausschwitzen des Stabilisators grundlegende Anforderungen an die Qualität eines Stabilisators darstellen.

In der vorliegenden Erfindung werden insbesondere Bernsteinsäureanhydride oder Derivate der Bernsteinsäure beschrieben, die ein oder mehrere sterisch gehinderte Mercaptophenolgruppen enthalten.

Die Erfindung betrifft Verbindungen der Formel I oder der Formel II

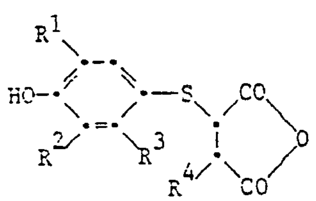 (I)

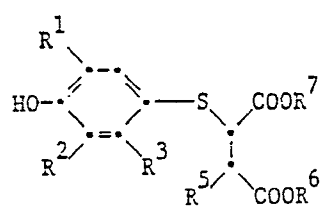 (II)

worin $R^1$ und $R^2$ unabhängig voneinander $C_1$-$C_{18}$ Alkyl, $C_5$-$C_6$ Cycloalkyl, Phenyl, das gegebenenfalls durch $C_1$-$C_{12}$ Alkyl substituiert ist, $C_7$-$C_9$ Aralkyl oder mit $C_1$-$C_{12}$ Alkyl substituiertes $C_7$-$C_9$ Aralkyl bedeuten, und worin $R^2$ zusätzlich Wasserstoff bedeutet, $R^3$ Wasserstoff oder Methyl ist, $R^4$ Wasserstoff oder $C_1$-$C_{16}$ Alkyl oder Phenyl ist, $R^5$ Wasserstoff, $C_1$-$C_6$ Alkyl, $C_1$-$C_{18}$ Alkylthio, Phenylthio oder ein Rest der Formel III bedeutet

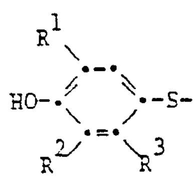 (III)

worin $R^1$, $R^2$ und $R^3$ die gleiche Bedeutung wie oben definiert besitzen ; und worin schliesslich $R^6$ und $R^7$ unabhängig voneinander Wasserstoff, $C_1$-$C_{30}$ Alkyl, durch $C_3$-$C_4$ Alkenoyloxy substituiertes $C_2$-$C_4$ Alkyl, Phenyl oder durch $C_1$-$C_{12}$ Alkyl substituiertes Phenyl sind.

$R^1$ und $R^2$ sind als geradkettiges oder verzweigtes $C_1$-$C_{18}$ Alkyl, beispielsweise Methyl, Ethyl, Isopropyl, n-Butyl, tert.-Butyl, tert.-Amyl, 1,1,3,3-Tetramethylbutyl, 2-Ethylhexyl, Dodecyl oder n-Octadecyl.

Bevorzugt werden $R^1$ und/oder $R^2$ als verzweigtes $C_4$-$C_8$ Alkyl, insbesondere als tert.-Butyl, tert.-Amyl oder 1,1,3,3-Tetramethylbutyl und besonders bevorzugt als tert.-Butyl.

$R^1$ und $R^2$ als $C_7$-$C_9$ Arylkyl sind beispielsweise Benzyl, α-Methylbenzyl oder α,α-Dimethylbenzyl.

Bevorzugt werden Verbindungen der Formel I oder der Formel II. $R^3$ ist vorzugsweise Wasserstoff.

Bevorzugt wird $R^4$ als Wasserstoff oder Methyl, insbesondere aber als Wasserstoff.

$R^5$ ist insbesondere Wasserstoff, Methyl oder eine Gruppe der Formel III

Die erfindungsgemässen Verbindungen lassen sich als Stabilisatoren für organisches Material wie beispielsweise Kunststoffe, Elastomere oder Harze verwenden.

Die Erfindung betrifft daher auch eine Zusammensetzung enthaltend ein gegen oxidativen, thermischen oder strahlungsinduzierten Abbau empfindliches organisches Material und mindestens eine Verbindung der Formel I und/oder der Formel II als Stabilisator, wobei als organisches Material vorzugsweise ein Polymer zu verstehen ist, besonders bevorzugt aber ein polyolefinisches Homo- oder Copolymer, insbesondere aber auch ein Homo-, Co- oder Terpolymer von Styrol.

Die Verbindungen der Formel I lassen sich insbesondere zur Stabilisierung der Homo- oder der Copolymeren von Polyolefinen einsetzen. Folgende polyolefinische Substrate lassen sich vorzugsweise mit den erfindungsgemässen Substanzen stabilisieren : Polyethylen, Polypropylen, Polyisobutylen, Poly-(buten-1), Poly-(penten-1), Poly-3(methylbuten-1), Poly-(4-methylpenten-1), unterschiedliche Ethylen-Propylen Copolymere, EPM, EPDM und ähnliche Verbindungen, Polystyrol und seine Co- oder Terpolymeren, wie beispielsweise schlagfestes Polystyrol, ABS-Harze, SBR, Polyisopren, Polybutadien, Nitilkautschuk, Naturkautschuk, oder Polyester wie beispielsweise Polyethylenterephthalat, Polybutylenterephthalat und deren Copolymere.

Ebenso lassen sich Polyurethane, Polycarbonate oder Polyamide wie beispielsweise Nylon 6, Nylon 6/6 oder dergleichen, sowie deren Copolymere oder Polysulfone mit den erfindungsgemässen Verbindungen stabilisieren.

Generell kann man die Stabilisatoren für folgende polymere Substrate verwenden :

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat ; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren ; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid ; sowie deren Copolymere wie Vinylchlorid-Vinylidenchlorid, Vinylchlorid-Vinylacetat oder Vinylidenchlorid-Vinylacetat.

8. Polymere, die sich von α,ß-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

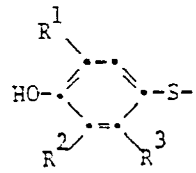

(III)

Besonders bevorzugt wird $R^5$ als Wasserstoff.

$R^6$ und/oder $R^7$ sind vorzugsweise Wasserstoff, $C_1$-$C_{18}$ Alkyl oder durch $C_3$-$C_4$ Alkenoyloxy substituiertes $C_2$-$C_4$ Alkyl.

Besonders bevorzugt werden $R^6$ und/oder $R^7$ als Wasserstoff, $C_1$-$C_{12}$ Alkyl oder durch $C_3$-$C_4$ Alkenoyloxy substituiertes Ethyl.

$R^6$ oder $R^7$ als $C_1$-$C_{30}$ Alkyl sind beispielsweise Methyl, Ethyl, n-Butyl, Amyl, 2-Ethylhexyl, Isooctyl, n-Dodecyl, n-Octadecyl, Eicosyl oder Triacontyl.

$R^6$ oder $R^7$ als durch Alkenoyloxy substituiertes Alkyl sind beispielsweise Ethyl, Propyl oder Butyl, bevorzugt Ethyl und sind mit Acryloyloxy, Methacryloyloxy oder Crotonyloxy substituiert, sind vorzugsweise jedoch mit Acryloyloxy oder Methacryloyloxy substituiert.

Die Bernsteinsäureanhydridderivate dieser Erfindung lassen sich durch Umsetzen eines geeigneten Mercaptophenols mit einem ungesättigten Anhydrid wie beispielsweise Maleinsäureanhydrid oder Citraconsäureanhydrid in Gegenwart eines Protonenakzeptors erhalten. Die Reaktion wird gegebenenfalls in einem Lösungsmittel durchgeführt. Es können aromatische Kohlenwasserstoffe wie beispielsweise Benzol, Toluol, Xylol und dergleichen, oder heterozyclische Ether wie beispielsweise Tetrahydrofuran als Lösungsmittel verwendet werden. Die Reaktionstemperatur umfasst das Intervall von Raumtemperatur bis zu 70 °C.

In einer bevorzugten Ausführungsform dieser Erfindung lässt man das Anhydrid mit dem Mercaptophenol in Gegenwart eines Protonenakzeptors wie beispielsweise einem tertiären Amin, bevorzugt Triethylamin oder Pyridin reagieren.

Die Bernsteinsäurederivate können aus den entsprechenden Hydroxyphenylthiobernsteinsäureanhydriden, deren Herstellung oben beschrieben wurde, erhalten werden, indem man die Anhydride mit einem geeigneten Alkohol oder mit einem substituierten Alkohol, beispielsweise mit 2-Hydroxyethylacrylat, oder mit Wasser oder mit einem Phenol umsetzt, wobei der Anhydridring geöffnet wird.

Man kann die Succinate aber auch auf direktem Wege erhalten, indem man die gleichen Reaktionsbedingungen wählt, wie zur Herstellung der Bernsteinsäureanhydride, und indem man ein geeignetes Mercaptophenol mit einem Dialkyl oder Monoalkylmaleat, -citraconat oder -acetylendicarboxylat umsetzt.

Wenn man ein Mercaptophenol mit einem Acetylendicarboxylat umsetzt, erhält man zwei Thiophenylreste in der Verbindung der Formel I, wobei der Substituent $R^5$ dann eine Gruppe der Formel III darstellt

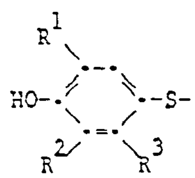

(III)

Ausgangsmaterialien zur Herstellung der erfindungsgemässen Bernsteinsäureanhydride oder Succinate sind im Handel erhältliche Produkte oder können nach bekannten Methoden hergestellt werden. Die Herstellung der Malonsäureester wird in Kirk-Othmer « Encyclopedia of Chemical Technology, 3rd Edit., Vol. 14, John Wiley, New York 1981, S. 776 » und in L.K. Flett und W.H. Gardener « Maleic Anhydride Derivatives, John Wiley, New York 1952 » beschrieben.

Als Mercaptophenole setzt man Substanzen der Formel IV ein

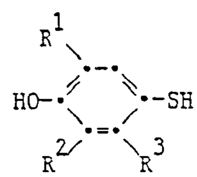

(IV)

worin R', $R^2$ und $R^3$ die gleiche Bedeutung wie oben beschrieben haben.

Bevorzugte Mercaptophenole dieses Typs sind 2,6-Di-tert.-butyl-4-mercaptophenol und 2-tert.-Butyl-6-methyl-4-mercaptophenol, sowie 2,6-Dimethyl-4-mercaptophenol und 2-tert.-Butyl-5-methyl-4-mercaptophenol.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z. B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z. B. von Bis-glycidylethern, von cycloaliphatischen Diepoxiden oder von aromatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z. B. Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.

29. Wässrige Emulsionen natürlicher oder synthetischer Kautschuke, wie z. B. Naturkautschuk-Latex oder Latices von carboxylierten Styrol-Butadien-Copolymeren.

Die Erfindung betrifft weiterhin ein Verfahren zum Stabilisieren von organischem Material gegen oxidativen, thermisch- oder strahlungs-induzierten Abbau, dadurch gekennzeichnet, dass man dem Material mindestens eine Verbindung der Formel I und/oder der Formel II wie oben beschrieben zusetzt.

Die erfindungsgemässen Stabilisatoren werden den Kunststoffen in Mengen von 0,01 bis 5 Gew.% bezogen auf das stabilisierte Material zugemischt. Je nach Verwendung und Substrat wird die Stabilisatormenge variiert. Bevorzugt verwendet man 0,05 bis 2 Gew.% des Stabilisators, besonders bevorzugt 0,1 bis 1 Gew.%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit einem pulverförmigen Polymeren gemischt werden, oder eine Emulsion oder eine Suspension des Stabilisators kann mit einer Lösung, Suspension oder Emulsion des Polymeren vermischt werden.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

Die stabilisierten Polymerzusammensetzungen können gegebenenfalls noch weitere Zusätze enthalten, wie zum Beispiel :

1. Antioxidantien

1.1 Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol

2,4,6-Tri-cyclohexylphenol
2,6-Di-tert.butyl-4-methoxymethylphenol

1.2 Alkylierte Hydrochinone
2,6-Di-tert.butyl-4-methoxyphenol
2,5-Di-tert.butyl-hydrochinon
2,5-Di-tert.amyl-hydrochinon
2,6-Diphenyl-4-octadecycloxyphenol
2,5-Di-tert.butyl-hydrochinon
3,5-Di-tert.butyl-4-hydroxyamid
Tris-(3,5-di tert.butyl-4-hydroxyphenyl)-phosphit
3,5-Di-tert.butyl-4-hydroxyphenylstearat
Bis-(3,5-di-tert.butyl-4-hydroxyphenyl)-adipat

1.3 Hydroxylierte Thiodiphenylether
2,2'-Thio-bis-(6-tert. butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)
4,4'-Thio-bis-(3,6-di-sek.amylphenol)
4,4'-Bis-(2,6-dimethyl-4-hydroxyphenyl)-disulfid

1.4 Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-ter.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.
1,1-Bis-(3,5-dimethyl-2-hydroxyphenyl)-butan
2,2-Bis-(3,5-di-tert.butyl-4-hydroxyphenyl)-propan
1,1,5,5-Tetra-(5-tert.butyl-4-hydroxy-2-methylphenyl)-pentan

1.5.0-, N- und S-Benzylverbindungen
3,3,5,5-Tetra-tert.butyl-4,4-dihydroxydibenzylether
Octacedyl-4-hydroxy-3,5-dimethylbenzylmercaptoacetat
Tris-(3,5-di-tert.butyl-4-hydroxybenzyl)-amin
Bis-(4-tert.butyl-3-hydroxy-2,6-dimethylbenzyl)-dithioterephthalat

1.6 Hydroxybenzylierte Malonate
Di-octadecyl-2,2-bis-(3,5-di-tert.butyl-2-hydroxybenzyl)-malonat
Di-octadecyl-2-(3-tert.butyl-4-hydroxy-5-methylbenzyl)-malonat
Di-dodecylmercaptoethyl-2,2-bis-(3,3-di-tert.butyl-4-hydroxybenzyl)-malonat
Di-[4-(1,1,3,3-tetramethylbutyl)-phenyl]-2,2-bis-(3,5-di-tert.butyl-4-hydroxybenzyl)-malonat

1.7 Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat

3,5-Di-tert.butyl-4-hydroxybenzyl-phosphonsäure-dioctadecylester
3,5-Di-tert.butyl-4-hydroxybenzyl-phosphonsäure-monoethylester, Calcium-salz
1,4-Bis-(3,5-di-tert.butyl-4-hydroxybenzyl)-2,3,5,6-tetramethylbenzol
2,4,6-Tris-(3,5-di-tert.butyl-4-hydroxybenzyl)-phenol.

1.8 s-Triazinverbindungen
2,4-Bis-octylmercapto-6-(3,5-di-tert.butyl-4-hydroxyanilino)-s-triazin
2-Octylmercapto-4,6-bis-(3,5-di-tert.butyl-4-hydroxyanilino)-s-triazin
2-Octylmercapto-4,6-bis-(3,5-di-tert.butyl-4-hydroxyphenoxy)-s-triazin
2,4,6-Tris-(3,4-di-tert.butyl-4-hydroxyphenylethyl)-s-triazin
1,3,5-Tris-(3,5-di-butyl-4-hydroxyphenylethyl)-s-triazin
1,3,5-Tris-(3,5-di-tert.butyl-4-hydroxybenzyl)-isocyanurat

1.9 Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.10 Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonandiol |
| Ethylenglykol | 1,2-Propandiol |
| Trimethylhexandiol | 3-Thia-pentadecanol |
| Trimethylolpropan | Trimethylolethan |

4-Hydroxymethyl-1-phospha-2,6,7-trioxabicyclo-[2,2,2]-octan

1.11 Ester der β(5-tert.butyl-4-hydroxy-3-methylphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonandiol |
| Ethylenglycol | 1,2-Propandiol |
| 3-Thia-undecanol | 3-Thia-pentadecanol |
| Trimethylolpropan | Trismethylolethan |

4-Hydroxy-1-phospha-2,6,7-trioxabicyclo-[2,2,2]-octan

1.12 Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin
N,N'-Di-(3,5-di-tert.butyl-4-hydroxyphenylpropionyl)-hexamethylendiamin
N,N'-Bis-(3,5-di-tert.butyl-4-hydroxyphenylpropionyl)-hydrazin

1.13 Ester der 3,5-Di-tert.butyl-4-hydroxyphenylessigsäure mit ein- oder mehrwertigen Alkoholen wie z. B. :

| | |
|---|---|
| Methanol | Ethanol |
| Octadecanol | 1,6-Hexandiol |
| 1,9-Nonandiol | Ethylenglycol |
| 1,2-Propandiol | Diethylenglycol |
| Thiodiglycol | Neopentylglycol |

Pentaerithrit
3-Thia-pentadecanol
Trimethylolethan
3-Thia-undecanol
Trimethylhexandiol
Trimethylolpropan

Tris-hydroxyethyl-isocyanurat
4-Hydroxymethyl-1-phospha-2,6,7-trioxabicyclo-[2,2,2]-octan
und besonders bevorzugt der Tetra-bis Ester mit Pentaerithrit

1.14 Benzylphosphonate wie zum Beispiel :
Dimethyl-3,5-di-tert.butyl-4-hydroxybenzyl-phosphonat
Diethyl-3,5-di-tert.butyl-4-hydroxybenzyl-phosphonat
Dioctadecyl-3,5-di-tert.butyl-4-hydroxybenzyl-phosphonat
Dioctadecyl-5-tert.butyl-4-hydroxy-3-methylbenzyl-phosphonat

Im Folgenden sind Beispiele von weiteren Additiven aufgezählt, die zusammen mit den oben erwähnten Antioxidantien und den erfindungsgemässen Stabilisatoren eingesetzt werden können. Zu diesen Stoffen zählen beispielsweise :

2. UV-Absorber und Lichtschutzmittel

2.1 2-(2'-Hydroxyphenyl)-benztriazole, wie z. B. das 5'-Methyl-, 3', 5'-Di-tert.butyl-, 5'-Tert.butyl-, 5'-(1,1,3,3-Tetramethylbutyl)-, 5-Chlor-3', 5-di-tert.butyl-, 5-Chlor-3'-tert.butyl-5'-methyl-, 3'-sec.Butyl-5'-tert.butyl, 4'-Octoxy-, 3',5'-Ditert.amyl-, 3',5'-Bis-(α,α-dimethylbenzyl)-, 3'- -Methylbenzyl-5'-methyl-5-chlor-, 4'-Hydroxy-, 4'-Methoxy-, 3'-Methyl-5'-carbomethoxyethyl-, 3',5'-Bis-(α,α-dimethylbenzyl)-5-chlor-, 3',5'-Di-tert.octylphenyl, 3',5'-Di-tert.-octylphenyl-5-chlor- oder 5-Chlor-3',5'-di-tert. amyl-Derivate.

2.2 2,4-Bis-(2-hydroxyphenyl)-6-alkyl-s-triazine wie beispielsweise das 6-Ethyl-, 6-Heptadecyl- oder das 6-Undecyl-Derivat.

2.3 2-Hydroxybenzophenone, wie z. B. das 4-Hydroxy-, 4-Methoxy-, 4-Octoxy-, 4-Decyloxy-, 4-Dodecyloxy-, 4-Benzyloxy, 4,2',4'-Trihydroxy, 2'-Hydroxy-4,4'-dimethoxy-Derivat.

2.4.1,3-Bis-(2'-hydroxybenzoyl)-benzole wie beispielsweise :
1,3-Bis-(2'-hydroxy-4'-hexyloxybenzoyl)-benzol
1,3-Bis-(2'-hydroxy-4'-octyloxybenzoyl)-benzol
1,3-Bis-(2'-hydroxy-4'-dodecyloxybenzoyl)-benzol

2.5 Ester von gegebenenfalls substituierten Benzoesäuren, wie z. B.
4-Tert.butyl-phenylsalicylat, Phenylsalicylat, Octylphenyl- salicylat, Dibenzoylresorcin, Bis-(4-tert.butylbenzoyl)-resorcin, Benzolresorcin, 3,5-Di-tert.butyl-4-hydroxybenzoesäure-2,4-ditert.butylphenylester, 3,5-Di-tert.butyl-4-hydroxybenzoesäurehexadecylester oder der n-Octadecylester oder der 2-Methyl-4,6-di-tert. butylester.

2.6 Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

2.7 Nickelverbindungen, wie z. B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.8 Sterisch gehinderte Amine, wie z. B. Bis-(2,2,6,6-tetramethylpiperidyl)-sebacat Bis-(1,2,2,6,6-pentamethylpiperidyl)-sebacat n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin, Tris-(2,2,6,6-tetramethylpiperidyl)-nitrilotriacetat, Tetrakis-(2,2,6,6-tetramethyl-4-piperidyl)-1,2,3,4-butantetracarbonsäure, 1,1'-(1,2-ethandiyl)-bis-(3,3,5,5-tetramethyl-piperazinon) 4-Benzoyl-2,2,6,6-tetramethylpiperidin, 4-Stearyl-oxy-2,2,6,6-tetramethylpiperidin, 3-n-Octyl-7,7,9,9-tetramethyl-1,3,8-triaza-spiro[4,5]decan-2,4-dion.

2.9 Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Isodecyloxy-2,4,8,10-tetraza-3,9-diphospha-spiro-[5,5]-undecan und Tri-(4-hydroxy-3,5-ditert.butylphenyl)-phosphit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Iodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert. Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

11. Aminoaryl Derivate wie beispielsweise : Phenyl-1-naphthylamin, Phenyl-2-naphthylamin, N,N'-Diphenyl-p-phenylendiamin, N,N'-Di-2-naphthyl-p-phenylendiamin, N,N'-Dinaphthyl-p-phenylendiamin, N,N'-Di-sek.butyl-p-phenylendiamin, 6-Ethoxy-2,2,4-trimethyl-1,2-dihydrochinolin, 6-Dodecyl-2,2,4-trimethyl-1,2-dihydrochinolin, Mono- und Dooctyliminodibenzyl, polymerisiertes 2,2,4-Trimethyl-1,2-dihydrochinolin, octyliertes Diphenylamin, nonyliertes Diphenylamin, N-Phenyl-N'-cyclohexyl-p-phenylendiamin, N-Phenyl-N'-isopropyl-p-phenylendiamin, N,N'-Di-sek.octyl-p-phenylendiamin, N-Phenyl-N'-sek.octyl-p-phenylendiamin, N,N'-Di-(1,4-dimethylpentyl)-p-phenylendiamin, N,N'-Di-(1,4-dimethylpentyl)-p-phenylendiamin, N,N'-Dimethyl-N,N'-di-(sek.octyl)-p-phenylendiamin, 2,6-Dimethyl-4-methoxyanilin, 4-Ethoxy-N-sek.butylanilin, Kondensationsprodukt von Diphenylamin und Aceton, Aldol-1-naphthylamin und Phenothiazin.

In einer besonders bevorzugten Ausführungsform werden die erfindungsgemässen Verbindungen als Stabilisatoren zusammen mit Dialkylthiodipropionaten wie beispielsweise Di-laurylthiodipropionat oder Di-stearylthiodipropionat eingesetzt.

Diese Kombinationen eignen sich besonders gut zur Stabilisierung von Polyolefinen insbesondere von Polypropylen.

Auch in Kombination mit organischen Phosphiten stellen die erfindungsgemässen Verbindungen besonders wirksame Stabilisatoren für organisches Material dar. Diese Stabilisatorkombination wird vorzugsweise bei Polyolefinen, wie beispielsweise Polyethylen und insbesondere Polypropylen, sowie bei Polystyrol und unterschiedlichsten Kohlenwasserstoffelastomeren angewendet.

Besonders wirksame organische Phosphite sind beispielsweise Triphenylphosphit, Tri-(nonylphenyl)-phosphit, Trilaurylphosphit, Trioctadecylphosphit, Distearyl-pentaerythritdiphosphit, Tri-(3,5-di-tert.-butyl-4-hydroxyphenyl)-phosphit, Tris-(2,4-di-tert.-butylphenyl)-phosphit, Diisodecylpentaerythrit-diphosphit, Di-(2,4-di-tert.-butylphenyl)-pentaerythritdiphosphit, 3,9-Di-n-octadecyloxy-2,4,8,10-tetraoxa-3,9-diphosphaspiro-[5,5]-undecan, 3,9-Bis-(2,4-di-tert.-butylphenyloxy)-2,4,8,10-tetraoxa-3,9-diphosphaspiro-[5,5]-undecan, 3,9-Isodecyloxy-2,4,8,10-tetraoxa-3,9-diphosphaspiro)-[5,5]-undecan, Tri-[2-(2,4,8,10-tetra-tert.-butyl-dibenzo[c,e]-1,3,2-dioxaphosphepan-6-yloxy)-ethyl]-amin, Tristearyl-sorbit-triphosphit oder Mischungen dieser Verbindungen.

Die erfindungsgemässen Verbindungen der Formel I oder der Formel II werden können aber auch als

alleiniger Stabilisator verwendet werden und wirken dann hauptsächlich als Antioxidans oder als Lichtschutzmittel oder sie wirken sowohl als Antioxidans und als Lichtschutzmittel.

Beispiel 1

Herstellung von Dimethyl-[2.3-bis-(3.5-di-tert.-butyl-4-hydroxyphenylthio)]-succinat

23,84 g 3.5-Di-tert.-butyl-4-mercaptophenol und 0,5 g Triethylamin werden mit100 ml Toluol in einem getrockneten 300 ml Kolben unter Stickstoff vorgelegt. Zu der Lösung tropft man langsam eine Lösung aus 7,11 g Dimethyl-acetyl-endicarboxylat in 50 ml Toluol, wobei die Temperatur unter 30 °C gehalten wird. Die Reaktionsmischung wird über Nacht gerührt, anschliessend das Lösungsmittel im Vakuum abgezogen und der Rückstand aus Heptan : Toluol = 1 : 1 umkristallisiert. Man erhält 22,06 g (Ausbeute 71 %) eines weissen Feststoffs. Das $^1$H-NMR-Spektrum ist in Uebereinstimmung mit dem threo-Isomeren.

Analyse :

| | | |
|---|---|---|
| berechnet | C 66,0 | H 8,1 |
| gefunden | C 66,3 | H 8,0 |

Beispiel 2

Herstellung von Dimethyl-2-(3,5-di-tert.-butyl-4-hydroxyphenylthio)-succinat

Das Verfahren von Beispiel 1 wird wiederholt, indem man 23,84 g 2,6-Di-tert.-butyl-4-mercaptophenol, 14,41 g Dimethylmaleat und 0,5 g Triethylamin miteinander umsetzt. Das Produkt wird aus Heptan umkristallisiert und man erhält 30,44 g (Ausbeute 80 %) eines weissen Feststoffs vom F.P. 94 °C bis 96 °C.

Analyse :

| | | |
|---|---|---|
| berechnet | C 62,8 | H 7,9 |
| gefunden | C 63,0 | H 8,0 |

Beispiel 3

Herstellung von Di-n-butyl-2-(3,5-di-tert.-butyl-4-hydroxyphenylthio)-succinat

Das Verfahren von Beispiel 1 wird wiederholt, indem man 11,92 g 2,6-Di-tert.-butyl-4-mercaptophenol, 11,41 g Di-n-butylmaleat und 0,5 g Triethylamin miteinander umsetzt. Das Produkt wird säulenchromatographisch gereinigt und man erhält eine klare syrupähnliche Substanz.

Analyse :

| | | |
|---|---|---|
| berechnet | C 66,9 | H 9,1 |
| gefunden | C 66,8 | H 8,7 |

Beispiel 4

Herstellung von Di-n-dodecyl-2-(3,5-di-tert.-butyl-4-hydroxyphenylthio)-succinat

Das Verfahren von Beispiel 1 wird wiederholt, indem man 7,15 g 2,6-Di-tert.-butyl-4-mercaptophenol, 13,58 g Di-n-dodecylmaleat und 0,3 g Triethylamin miteinander umsetzt. Das Produkt wird säulenchromatographisch gereinigt und man erhält eine klare Flüssigkeit.

Analyse :

| | | |
|---|---|---|
| berechnet | C 73,0 | H 10,8 |
| gefunden | C 73,0 | H 10,8 |

Beispiel 5

Herstellung von 2-(3,5-Di-tert.-butyl-4-hydroxyphenylthio)-bernsteinsäureanhydrid

In Analogie zum Verfahren von Beispiel 1 verwendet man 23,84 g 2,6-Di-tert.-butyl-4-mercaptophenol, 9,8 g Maleinsäureanhydrid und 0,51 g Triethylamin. Der Rückstand wird einmal aus Hexan/Toluol und einmal aus Cyclohexan umkristallisiert und man erhält 19,0 g eines gelbstichigen Feststoffs vom F.P. 92 °C bis 93 °C.

Analyse :

| | | |
|---|---|---|
| berechnet | C 64,3 | H 7,2 |
| gefunden | C 64,2 | H 7,5 |

Beispiel 6

Herstellung des 2-Acryloyloxyethylmonoesters der 2-(3,5-Di-tert.-butyl-4-hydroxyphenylthio)-bernsteinsäure

Eine Mischung aus 8,41 g 2-(3,5-Di-tert.-butyl-4-hydroxyphenylthio)-bernsteinsäureanhydrid und 2,90 g 2-Hydroxyethylacrylat wird unter Stickstoff eine Stunde lang auf 70 °C bis 80 °C erhitzt. Der Rückstand wird zweimal aus einer Toluol/Petrolethermischung umkristallisiert und man erhält 3,86 g einer weissen Festsubstanz vom F.P. 89 °C bis 91 °C.

Analyse :
berechnet S 7,1
gefunden S 7,0

Beispiel 7

In diesem Beispiel wird die stabilisierende Wirkung der erfindungsgemässen Verbindungen in schlagfestem Polystyrol demonstriert.

a) Herstellung der Proben : Man stellt eine Lösung von 8 Gew.-% Polybutadien-Kautschuk (Firestone DIENE 55) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, das mit einem Wendelrührer ausgerüstet ist. Da die meisten IPS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121 °C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt : eine Stunde bei 100 °C um die Anfangstemperatur vorzugeben, eine weitere Stunde, um 140 °C zu erreichen und anschliessend weitere 8 Stunden, wobei die Temperatur jede Stunde um 10 °C erhöht wird, bis schliesslich ein Maximum von 220 °C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200 °C für 45 Minuten bei einem Druck von 1,33 mbar belassen, um flüchtige Bestandteile zu entfernen. Nach dieser Behandlung wird der Block sofort in einer heizbaren hydraulischen Presse bei 205 °C zwischen zwei Aluminiumfolien zu einem dicken Stab gepresst (drei Minuten Erhitzen, fünf Minuten ungeheizt). Der Stab wird mittels einer Handsäge zerkleinert und anschliessend granuliert. Alle Polymermischung werden bei 205 °C extrudiert und dann zerkleinert. Die Pellets werden unter Druck bei 205 °C in dehnbare 3,175 mm dicke Streifen verformt.

b) Testverfahren : Diese Probestreifen werden dann bei 150 °C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80 °C im Ofen gealtert. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts : Ziehgeschwindigkeit : 5 mm/Minute).

In der folgenden Tabelle Ia sind die Ergebnisse der Dehnbarkeitsmessungen und der Messungen des Yellowness Indexes von bei 80 °C ofengealterten Proben angegeben, während Tabelle 1b) Minuten von 150 °C ofengealterten Proben enthält.

(Siehe Tabelle 1a Seite 12 f.)

Tabelle 1a

Dehnbarkeitsmessungen und Yellowness Index von bei 80 °C ofengealterten Proben mit und ohne Stabilisator

| Additiv | Stabilisatormenge (Gew.%) | Dehnbarkeit der Probe (%) und Dauer der Hitzebelastung (h) | | | | |
|---|---|---|---|---|---|---|
| | | 0 h | 300 h | 600 h | 900 h | 1200 h |
| Produkt von Beispiel 5 | 0,1 | 50 | 49 | 28 | 12 | 7 |
| - | - | 33 | 9 | 3 | 3 | 3 |

| Additiv | Stabilisatormenge (Gew.%) | Yellowness Index und Dauer der Hitzebelastung (h) | | | | |
|---|---|---|---|---|---|---|
| | | 0 h | 300 h | 300 h | 900 h | 1200 h |
| Produkt von Beispiel 5 | 0,1 | -3 | 0 | 0 | 2 | 7 |
| - | - | 7 | 14 | 45 | 59 | - |

Tabelle 1b

Dehnbarkeitsmessungen und Yellowness Index von bei 150 °C ofengealterten Proben mit und ohne Stabilisator

| Additiv | Stabilisator-Menge (Gew.%) | Dehnbarkeit der Probe (%) und Dauer der Hitzebelastung (h) | | | |
|---|---|---|---|---|---|
| | | 0 h | 0,5 h | 1 h | 1,5 h |
| Produkt von Beispiel 5 | 0,1 | 50 | 23 | 13 | 3 |
| - | - | 33 | 7 | 7 | 3 |

| Additiv | Stabilisatormenge (Gew.%) | Yellowness Index und Dauer der Hitzebelastung (h) | | | |
|---|---|---|---|---|---|
| | | 0 h | 0,5 h | 1 h | 1,5 h |
| Produkt von Beispiel 5 | 0,1 | -3 | 1 | 12 | 22 |
| - | - | 7 | 18 | 30 | 38 |

Beispiel 8

Lichtstabilität von Polypropylen

Unstabilisiertes Polypropylenpulver (Hercules Profax 6501) wird mit einer bestimmten Menge eines Additivs gemischt. Diese Mischungen werden anschliessend bei 182 °C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei

220 °C und 12 bar zu einer 0,127 mm dicken Probe verformt. Diese Probe wird ultraviolettem Licht bis zur Zersetzung ausgesetzt. Als Zersetzungszeitpunkt wird diejenige Anzahl von Stunden gewertet, bei der die IR-spektroskopisch gemessene dekadische Extinktion der Carbonylbande 50 % des Anfangswertes erreicht. Die Messdaten sind in der folgenden Tabelle 2 dargestellt :

Tabelle 2

| Additiv | Stabilisatormenge (Gew.%) | Zahl der Stunden bis zur Zersetzung der bestrahlten Proben |
|---|---|---|
| Produkt von Beispiel 2 | 0,2 | 360 |
| Produkt von Beispiel 3 | 0,2 | 400 |
| Produkt von Beispiel 5 | 0,2 | 420 |
| - | - | 200-300 |

Beispiel 9

Stabilisatorwirkung einer Kombination der erfindungsgemässen Verbindungen mit DSTDP in Polypropylen

Unstabilisiertes Polypropylenpulver (Hercules Profax 6501) wird mit 0,2 Gew.-% des jeweiligen Stabilisators gemischt. Zusätzlich werden Polypropylenproben hergestellt, die 0,1 Gew.-% einer erfindungsgemässen Verbindung und 0,3 Gew.-% Distearyl-β-thiodipropionat (DSTDP) enthalten.

Diese Mischungen werden anschliessend bei 182 °C 10 Minuten lang auf einem Mischwalzwerk plastifiziert. Anschliessend werden die Polypropylenfolien von der Walze entfernt und erkalten gelassen.

Die so vorbehandelten Proben werden zerkleinert und anschliessend 7 Minuten lang in einer hydraulischen Presse bei 218 °C und unter einem Druck von 19,25 kg/cm$^2$ zu Platten von 0,64 mm Dicke verformt. Diese Platten werden bei 150 °C in einem Umluftofen gealtert. Als Zersetzungszeitpunkt wird diejenige Anzahl von Stunden gewertet, nach der die Proben erste äusserliche Anzeichen eines Zerfalls (braune Ecken, Risse) zeigen.

Die Ergebnisse sind in der untenstehenden Tabelle aufgelistet.

| Stabilisator | Stabilisatormenge (Gew.%) | Zersetzungszeit (Stunden) |
|---|---|---|
| - | - | < 3 |
| DSTDP | 0,3 | <20 |
| Produkt von Beispiel 2 | 0,2 | < 24 |
| Produkt von Beispiel 2 + DSTDP | 0,1 + 0,3 | < 24 |
| Produkt von Beispiel 3 | 0,2 | < 24 |
| Produkt von Beispiel 3 + DSTDP | 0,1 + 0,3 | < 24 |
| Produkt von Beispiel 4 | 0,2 | 420 |
| Produkt von Beispiel 4 + DSTDP | 0,1 + 0,3 | 720 |
| Produkt von Beispiel 5 | 0,2 | 20 |
| Produkt von Beispiel 5 + DSTDP | 0,1 + 0,3 | 90 |

Die Produkte von Beispielen 2 und 3 besitzen ein relativ niedriges Molekulargewicht und verflüchtigen sich teilweise während der Testprozedur bei 150 °C. Die Verbindung von Beispiel 4 besitzt ein höheres Molekulargewicht und verbleibt während des Tests in der Polypropylenprobe.

**Patentansprüche**

1. Verbindungen der Formel I oder der Formel II

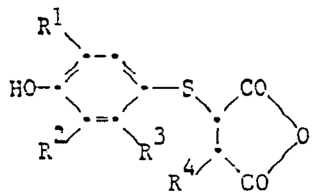 (I) 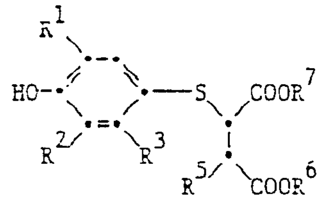 (II)

worin
$R^1$ und $R^2$ unabhängig voneinander $C_1$-$C_{18}$ Alkyl, $C_5$-$C_6$ Cycloalkyl, Phenyl, das gegebenenfalls durch $C_1$-$C_{12}$ Alkyl substituiert ist, $C_7$-$C_9$ Aralkyl oder mit $C_1$-$C_{12}$ Alkyl substituiertes $C_7$-$C_9$ Aralkyl bedeuten, und worin $R^2$ zusätzlich Wasserstoff bedeutet,
$R^3$ Wasserstoff oder Methyl ist,
$R^4$ Wasserstoff oder $C_1$-$C_6$ Alkyl oder Phenyl ist,
$R^5$ Wasserstoff, $C_1$-$C_6$ Alkyl, $C_1$-$C_{18}$ Alkylthio, Phenylthio oder ein Rest der Formel III bedeutet

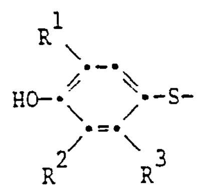 (III)

worin $R^1$, $R^2$ und $R^3$ die gleiche Bedeutung wie oben definiert besitzen ; und worin schliesslich $R^6$ und $R^7$ unabhängig voneinander Wasserstoff, $C_1$-$C_{30}$ Alkyl, durch $C_3$-$C_4$ Alkenoyloxy substituiertes $C_2$-$C_4$ Alkyl, Phenyl oder durch $C_1$-$C_{12}$ Alkyl substituiertes Phenyl sind.

2. Verbindungen der Formel I oder der Formel II gemäss Anspruch 1, worin $R^1$ und/oder $R^2$ verzweigtes $C_4$-$C_8$ Alkyl sind.

3. Verbindungen der Formel I oder der Formel II gemäss Anspruch 2, worin $R^1$ und $R^2$ tert.-Butyl bedeuten.

4. Verbindungen der Formel I oder der Formel II gemäss Anspruch 1, worin $R^3$ Wasserstoff ist.

5. Verbindungen der Formel I oder der Formel II gemäss Anspruch 1, worin $R^4$ Wasserstoff oder Methyl ist.

6. Verbindungen der Formel I oder der Formel II gemäss Anspruch 1, worin $R^4$ Wasserstoff bedeutet.

7. Verbindungen der Formel I oder der Formel II gemäss Anspruch 1, worin $R^5$ Wasserstoff, Methyl oder eine Gruppe der Formel III bedeutet.

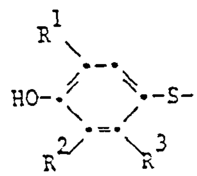 (III)

und worin $R^1$, $R^2$ und $R^3$ die gleiche Bedeutung besitzen wie in Anspruch 1 definiert.

8. Verbindungen der Formel I oder der Formel II gemäss Anspruch 1, worin $R^5$ Wasserstoff bedeutet.

9. Verbindungen der Formel I oder der Formel II gemäss Anspruch 1, worin $R^6$ und $R^7$ unabhängig voneinander Wasserstoff, $C_1$-$C_{18}$ Alkyl oder durch $C_3$-$C_4$ Alkenoyloxy substituiertes $C_2$-$C_4$ Alkyl bedeuten.

10. Verbindungen der Formel I oder der Formel II gemäss Anspruch 1, worin $R^6$ und $R^7$ unabhängig voneinander Wasserstoff, $C_1$-$C_{12}$ Alkyl oder durch $C_3$-$C_4$ Alkenyloxy substituiertes Ethyl bedeuten.

11. Die Verbindung Dimethyl-[2,3-bis-(3,5-di-tert.-butyl-4-hydroxyphenylthio)]-succinat gemäss Anspruch 1.

12. Die Verbindung Dimethyl-2-(3,5-di-tert.-butyl-4-hydroxyphenylthio)-succinat gemäss Anspruch 1.

13. Die Verbindung Di-n-butyl-2-(3,5-di-tert.-butyl-4-hydroxyphenylthio)-succinat gemäss Anspruch 1.

14. Die Verbindung Di-n-dodecyl-2-(3,5-di-tert.-butyl-4-hydroxyphenylthio)-succinat gemäss Anspruch 1.

15. Die Verbindung 2-(3,5-Di-tert.-butyl-4-hydroxyphenylthio)-bernsteinsäureanhydrid gemäss Anspruch 1.

16. Die Verbindung 2-(3,5-Di-tert.-butyl-4-hydroxyphenylthio)-bernsteinsäuremono-2-acryloyloxyethylester gemäss Anspruch 1.

17. Zusammensetzung enthaltend ein gegen oxidativen, thermischen oder strahlungsinduzierten Abbau empfindliches organisches Material und mindestens eine Verbindung der Formel I und/oder der Formel II gemäss Anspruch 1 als Stabilisator.

18. Zusammensetzung gemäss Anspruch 17, worin das organische Material ein Polymer ist.

19. Zusammensetzung gemäss Anspruch 18, worin das Polymer ein Homo- oder Copolymer eines Polyolefins bedeutet.

20. Zusammensetzung gemäss Anspruch 18, worin das Polymer ein Homo-, Co- oder Terpolymer von Styrol bedeutet.

21. Zusammensetzung gemäss Anspruch 17, die zusätzlich ein Dialkylthiodipropionat als Coadditiv enthält.

22. Zusammensetzung gemäss Anspruch 17, die zusätzlich ein organisches Phosphit als Coadditiv enthält.

23. Zusammensetzung gemäss Anspruch 22, worin das organische Phosphit, Triphenylphosphit, Tri-(nonylphenyl)-phosphit, Trilaurylphosphit, Trioctadecylphosphit, Distearyl-pentaerythritdiphosphit, Tris-(3,5-ditert.-butyl-4-hydroxyphenyl)-phosphit, Tris-(2,4-di-tert.-butylphenyl)-phosphit, Diisodecylpentaerythrit-diphosphit, Di-(2,4-di-tert.-butylphenyl)-pentaerythrit-diphosphit, 3,9-Di-n-octadecyloxy-2,4,8,10-tetraoxa-3,9-diphosphaspiro-[5,5]-undecan, 3,9-Bis-(2,4-di-tert.-butylphenyloxy)-2,4,8,10-tetraoxa-3,9-diphosphaspiro-[5,5]-undecan, 3,9-Isodecyloxy-2,4,8,10-tetraoxa-3,9-diphosphaspiro-[5,5]-undecan, Tri-[2-(4,8,10-tetra-tert.-butyl-dibenzo-[c,e]-1,3,2-dioxaphosphepan-6-yloxy)-ethyl]-amin oder Tristearyl-sorbit-triphosphit bedeutet, oder worin die oben erwähnten Phosphite als Mischungen verwendet werden.

24. Verfahren zum Stabilisieren von organischem Material gegen oxidativen, thermisch- oder strahlungsinduzierten Abbau, dadurch gekennzeichnet, dass man dem Material mindestens eine Verbindung der Formel I und/oder der Formel II gemäss Anspruch 1 zusetzt.

## Claims

1. A compound of formula I or II

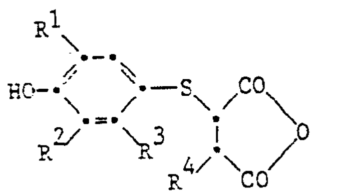 (I)

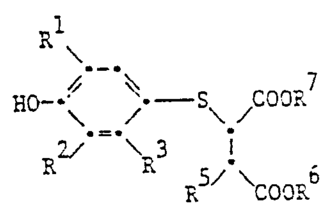 (II)

wherein

$R^1$ and $R^2$ are each independently $C_1$-$C_{18}$ alkyl, $C_5$-$C_6$ cycloalkyl, phenyl or phenyl substituted by $C_1$-$C_{12}$ alkyl, $C_7$-$C_9$ aralkyl which is unsubstituted or substituted by $C_1$-$C_{12}$ alkyl : $R^2$ may also be hydrogen ;

$R^3$ is hydrogen or methyl ;

$R^4$ is hydrogen, $C_1$-$C_6$ alkyl or phenyl ;

$R^5$ is hydrogen, $C_1$-$C_6$ alkyl, $C_1$-$C_{18}$ alkylthio, phenylthio, or a radical of formula III

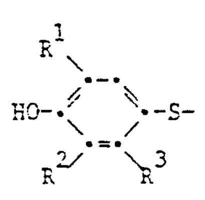 (III)

wherein $R^1$, $R^2$ and $R^3$ are as defined above ; and $R^6$ and $R^7$ are each independently hydrogen, $C_1$-$C_{30}$ alkyl, $C_2$-$C_4$ alkyl which is substituted by $C_3$-$C_4$ alkenoyloxy, phenyl or phenyl substituted by $C_1$-$C_{12}$ alkyl.

2. A compound of formula I or II according to claim 1, wherein $R^1$ and/or $R^2$ are branched-chain $C_4$-$C_8$ alkyl.

3. A compound of formula I or II according to claim 2, wherein $R^1$ and $R^2$ are each tert-butyl.

4. A compound of formula I or II according to claim 1, wherein $R^3$ is hydrogen.

5. A compound of formula I or II according to claim 1, wherein $R^4$ is hydrogen or methyl.

6. A compound of formula I or II according to claim 5, wherein $R^4$ is hydrogen.

7. A compound of formula I or II according to claim 1, wherein $R^5$ is hydrogen, methyl or a group of formula III

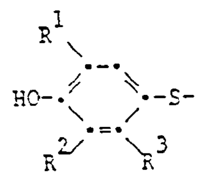

(III)

wherein $R^1$, $R^2$ and $R^3$ are defined as in claim 1.

8. A compound of formula I or II according to claim 1, wherein $R^5$ is hydrogen.

9. A compound of formula I or II according to claim 1, wherein $R^6$ and $R^7$ are each independently hydrogen, $C_1$-$C_{18}$ alkyl or $C_2$-$C_4$ alkyl which is substituted by $C_2$-$C_4$ alkenoyloxy.

10. A compound of formula I or II according to claim 9, wherein $R^6$ and $R^7$ are each independently hydrogen, $C_1$-$C_{12}$ alkyl or ethyl substituted by $C_3$-$C_4$ alkenoyloxy.

11. The compound, dimethyl 2,3-bis-(3,5-di-tert-butyl-4-hydroxyphenylthio)succinate, according to claim 1.

12. The compound, dimethyl 2-(3,5-di-tert-butyl-4-hydroxyphenylthio)succinate, according to claim 1.

13. The compound, di-n-butyl 2-(3,5-di-tert-butyl-4-hydroxyphenylthio)succinate, according to claim 1.

14. The compound, di-n-dodecyl 2-(3,5-di-tert-butyl-4-hydroxyphenylthio)succinate, according to claim 1.

15. The compound, 2-(3,5-di-tert-butyl-4-hydroxyphenylthio)succinate anhydride, according to claim 1.

16. The compound 2-(3,5-di-tert-butyl-4-hydroxyphenylthio)succinic acid 2-acryloyloxyethyl monoester, according to claim 1.

17. A composition of matter comprising an organic material subject to oxidative, thermal or actinic-induced degradation stabilized with an effective stabilizing amount of a compound of formula I or II according to claim 1.

18. A composition according to claim 17, wherein the organic material is a polymer.

19. A composition according to claim 18, wherein the polymer is a polyolefin homopolymer or copolymer.

20. A composition according to claim 18, wherein the polymer is a styrene homopolymer, copolymer or terpolymer.

21. A composition according to claim 17, which also contains an effective stabilizing amount of a coadditive which is a dialkyl thiodipropionate.

22. A composition according to claim 17 which also contains an effective stabilizing amount of a coadditive which is an organic phosphite.

23. A composition according to claim 22, wherein the organic phosphite is triphenylphosphite, tri(nonylphenyl) phosphite, trilauryl phosphite, trioctadecylphenylphosphite, di-stearyl-pentaerythrit-diphosphite, tris(3,5-di-tert.-butyl-4-hydroxyphenyl) phosphite, tri-(2,4-di-tert.-butylphenyl)-phosphite, diisodecylpentaerythrit-diphosphite, di-(2,4-di-tert.-butylphenyl)-pentaerythrit-diphosphite, 3,9-di-n-octadecyloxy-2,4,8,10-tetraoxa-3,9-diphosphaspiro [5.5]-undecan, 3,9-bis(2,4-di-tert.-butylphenyloxy)-2,4,8,10-tetraoxa-3,9-diphosphaspiro [5.5] undecane, 3,9-isodecyloxy-2,4,8,10-tetraaza-3,9-diphosphaspiro-[5.5]-undecane, tri [2,4,8,10-tetra-tert-butyl-dibenzo [c,e]-1,3,2-dioxaphosphepan-6-yloxy)ethyl] amine or tristearyl sorbitol triphosphite, or wherein the above phosphites are employed as mixtures.

24. A method of stabilizing organic material against oxidative, thermal or actinic-induced degradation, which comprises incorporating into said organic material an effective stabilizing amount of a compound of formula I or II according to claim 1.

## Revendications

1. Composés répondant à l'une des formules I et II :

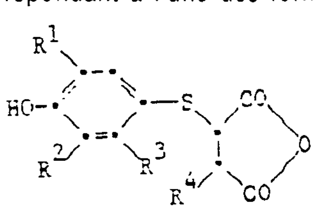

(I)

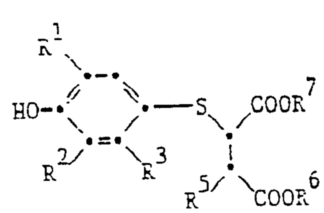

(II)

dans lesquelles

$R^1$ et $R^2$ représentent chacun, indépendamment l'un de l'autre, un alkyle en $C_1$-$C_{18}$, un cycloalkyle en $C_5$ ou $C_6$, un phényle éventuellement porteur d'un alkyle en $C_1$-$C_{12}$, un aralkyle en $C_7$-$C_9$ ou un aralkyle en $C_7$-$C_9$ éventuellement porteur d'un alkyle en $C_1$-$C_{12}$, le symbole $R_2$ pouvant en outre représenter l'hydrogène,

$R^3$ représente l'hydrogène ou un méthyle,

$R^4$ représente l'hydrogène, un alkyle en $C_1$-$C_6$ ou un phényle,

$R_5$ représente l'hydrogène, un alkyle en $C_1$-$C_6$, un alkylthio en $C_1$-$C_{18}$, un phénylthio ou un radical de formule III :

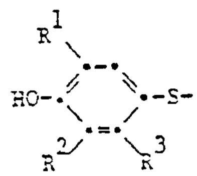

(III)

dans lequel $R^1$, $R^2$ et $R^3$ ont les mêmes significations de ci-dessus, et $R^6$ et $R^7$ représentent chacun, indépendamment l'un de l'autre, l'hydrogène, un alkyle en $C_1$-$C_{30}$, un alkyle en $C_2$-$C_4$ porteur d'un alcénoyloxy en $C_3$ ou $C_4$, un phényle ou un phényle porteur d'un alkyle en $C_1$-$C_{12}$.

2. Composés de formule I ou de formule II selon la revendication 1 dans lesquels $R^1$ et/ou $R^2$ représentent un alkyle en $C_4$-$C_8$.

3. Composés de formule I ou de formule II selon la revendication 2, dans lesquels $R^1$ et $R^2$ représentent un butyle tertiaire.

4. Composés de formule I ou de formule II selon la revendication 1, dans lesquels $R^3$ représente l'hydrogène.

5. Composés de formule I ou de formule II selon la revendication 1, dans lesquels $R^4$ représente l'hydrogène ou un méthyle.

6. Composés de formule I ou de formule II selon la revendication 1, dans lesquels $R^4$ représente l'hydrogène.

7. Composés de formule I ou de formule II selon la revendication 1, dans lesquels $R^5$ représente l'hydrogène, un méthyle ou un radical de formule III :

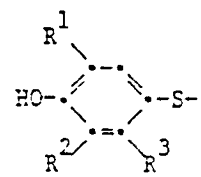

(III)

et les symboles $R^1$, $R^2$ et $R^3$ ont les mêmes significations que dans la revendication 1.

8. Composés de formule I ou de formule II selon la revendication 1, dans lesquels $R^5$ représente l'hydrogène.

9. Composés de formule I ou de formule II selon la revendication 1, dans lesquels $R^6$ et $R^7$ représentent chacun, indépendamment l'un de l'autre, l'hydrogène, un alkyle en $C_1$-$C_{18}$ ou un alkyle en $C_2$-$C_4$ porteur d'un alcénoyloxy en $C_3$ ou $C_4$.

10. Composés de formule I ou de formule II selon la revendication 1, dans lesquels $R^6$ et $R^7$ représentent chacun, indépendamment l'un de l'autre, l'hydrogène, un alkyle en $C_1$-$C_{12}$ ou un éthyle porteur d'un alcénoyloxy en $C_3$ ou $C_4$.

11. Composé selon la revendication 1, en l'espèce le bis-(ditert-butyl-3,5 hydroxy-4 phénylthio)-2,3 succinate de diméthyle.

12. Composé selon la revendication 1, en l'espèce le (ditert-butyl-3,5 hydroxy-4 phénylthio)-2 succinate de diméthyle.

13. Composé selon la revendication 1, en l'espèce le (ditert-butyl-3,5 hydroxy-4 phénylthio)-2 succinate de di-n-butyle.

14. Composé selon la revendication 1, en l'espèce le (ditert-butyl-3,5 hydroxy-4 phénylthio)-2 succinate de di-n-dodécyle.

15. Composé selon la revendication 1, en l'espèce l'anhydride (ditert-butyl-3,5 hydroxy-4 phénylthio)-2 succinique.

16. Composé selon la revendication 1, en l'espèce le (ditert-butyl-3,5 hydroxy-4 phénylthio)-2 succinate de mono-(acryloyloxy-2 éthyle).

17. Composition contenant une matière organique sensible à l'action destructrice de l'oxydation, de la chaleur ou du rayonnement et au moins un composé de formule I et/ou de formule II selon la revendication 1, jouant le rôle de stabilisant.

18. Composition selon la revendication 17 dans laquelle la matière organique est un polymère.

19. Composition selon la revendication 18 dans laquelle le polymère est un homopolymère ou un copolymère d'une polyoléfine.

20. Composition selon la revendication 18 dans laquelle le polymère est un homo-, co- ou terpolymère du styrène.

21. Composition selon la revendication 17 qui contient en outre, comme co-additif, un thiodipropionate de dialkyle.

22. Composition selon la revendication 17 qui contient en outre, comme co-additif, un phosphite organique.

23. Composition selon la revendication 22 dans laquelle le phosphite organique est le phosphite de triphényle, le phosphite de tris-(nonylphényle), le phosphite de trilauryle, le phosphite de tri-octadécyle, le distéaryloxy-3,9 tétraoxa-2,4,8,10 diphospha-3,9 spiro [5.5] undécane, le phosphite de tris-(ditertbutyl-3,5 hydroxy-4 phényle), le phosphite de tris-(ditert-butyl-2,4 phényle) ; le di-isodécyloxy-3,9 tétraoxa-2,4,8,10 diphospha-3,9 spiro [5.5] undécane, le bis-(ditert-butyl-2,4 phényloxy)-3,9 tétraoxa-2,4,8,10 diphospha-3,9 spiro [5.5] undécane, le di-n-octadécyloxy-3,9 tétraoxa-2,4,8,10 diphospha-3,9 spiro [5.5] undécane, le bis-(ditert-butyl-2,4 phényloxy)-3,9 tétraza-2,4,8,10 diphospha-3,9 spiro [5.5] undécane, le di-isodécyloxy-3,9 tétraza-2,4,8,10 diphospha-3,9 spiro [5.5] undécane, la tris-[(tétra-tert-butyl-2,4,8,10 dibenzo [c,e] dioxaphosphépanne-1,3,2 yl-6 oxy)-2 éthyl]-amine ou le tris-(stéaryl-phosphite) de sorbitol, ou dans laquelle les phosphites qui viennent d'être cités sont utilisés sous la forme de mélanges.

24. Procédé pour stabiliser une matière organique contre la dégradation par oxydation, sous l'action de la chaleur ou sous l'action d'un rayonnement, procédé caractérisé en ce qu'on ajoute à la matière au moins un composé de formule I et/ou de formule II selon la revendication 1.